# EUROPEAN PATENT APPLICATION

(11) **EP 4 474 387 A1**
(43) Date of publication of application: **11.12.2024**
(21) Application number: 23749879.5
(22) Date of filing: 06.02.2023
(51) Int. Cl.: C07H 21/00, C07H 1/02, C07H 21/02, C07H 21/04

(54) **METHOD FOR PRODUCING OLIGONUCLEOTIDE**

(30) Priority: 04.02.2022 JP 2022016004; 07.09.2022 JP 2022142047
(71) Applicant: Nitto Denko Corporation, Ibaraki-shi, Osaka 567-8680 (JP)
(72) Inventor: YOSHIDA, Kei, Ibaraki-shi, Osaka 567-8680 (JP); IIDA, Tomoyoshi, Ibaraki-shi, Osaka 567-8680 (JP); IWAMOTO, Masafumi, Ibaraki-shi, Osaka 567-8680 (JP); MAETA, Eri, Ibaraki-shi, Osaka 567-8680 (JP); MATSUNAMI, Jun, Ibaraki-shi, Osaka 567-8680 (JP)
(74) Representative: Hoffmann Eitle
(86) International application number: PCT/JP2023/003685
(87) International publication number: WO 2023/149563

(57) **Abstract**

The purpose of the present invention is to provide a method for suppressing the amount of nucleoside phosphoramidites remaining in a reactor and reducing the amount of nucleoside phosphoramidites used in a synthesis step in a method for producing an oligonucleotide. The above problem was solved by a method for producing an oligonucleotide, wherein: the method includes a step for binding a nucleoside phosphoramidite to a hydroxyl group, thiol group, or amino group at the 3' or 5' position of a nucleoside supported directly or indirectly on a carrier in the presence of an activating agent; and the amount of activating agent used in the aforementioned step is a 10.0 to 15.0-fold equivalent of the amount of nucleoside phosphoramidite used in said step.

## Description

### [Technical Field]

The present invention relates to a method for producing an oligonucleotide.

### [Background Art]

For chemical synthesis of nucleic acids such as DNA oligonucleotides and RNA oligonucleotides, the phosphoramidite method is widely used. In the phosphoramidite method, an oligonucleotide is typically synthesized through sequential addition of nucleoside phosphoramidites to a nucleoside, a nucleotide or an oligonucleotide in the presence of a suitable activator.

In the phosphoramidite method, nucleoside phosphoramidites are generally used in an amount that is 1.5 to 10.0 times the theoretical amount. Nucleoside phosphoramidites are expensive, of the raw synthesis materials, and thus, when the used amount of the nucleoside phosphoramidites can be reduced, a significant reduction in the production cost is expected.

However, no method for obtaining an oligonucleotide with a favorable purity while reducing the used amount of the nucleoside phosphoramidites has been known so far.

Patent Literature 1 discloses that the amount of the activator used is suitably 1 to 20 times the amount of the nucleoside derivative, preferably 1 to 10 times the molar quantity, but does not describe any method for obtaining an oligonucleotide with a favorable purity while suppressing the amount of the nucleoside phosphoramidites remaining in the reaction vessel in the synthesis step and reducing the used amount of the nucleoside phosphoramidites.

### [Prior Art Literature]

### [Patent Literature]

Patent Literature 1: WO2006/022323A

### [Summary of Invention]

### [Problem to be solved by the invention]

An object of the invention is to provide a method for suppressing the amount of the nucleoside phosphoramidites remaining in the reaction vessel and reducing the used amount of the nucleoside phosphoramidites in a method for producing an oligonucleotide.

### [Means of solving the problems]

While the present inventors have worked on extensive research on a method for producing an oligonucleotide, the inventors have found that the amount of the nucleoside phosphoramidites remaining in the reaction vessel in the synthesis step can be suppressed, and the used amount of the nucleoside phosphoramidites can be reduced by increasing the used amount of the activator. As a result of further continuous research based on the findings, the invention has been completed.

That is, the invention relates to the followings.
[1] A method for producing an oligonucleotide comprising a step of binding a nucleoside phosphoramidite(s) to a hydroxy group, a thiol group or an amino group at the 3' position or the 5' position of a nucleoside(s) which is directly or indirectly attached to supports in the presence of an activator(s), wherein the amount of the activator(s) used in the step is 10.0 to 15.0 equivalents of the amount of the nucleoside phosphoramidite(s) used in the step.
[2] The method according to [1], wherein the amount of the activator(s) used in the step is 10.0 to 25.0 equivalents of the nucleoside(s) attached to supports.
[3] The method according to [1] or [2], wherein the amount of the nucleoside phosphoramidite(s) used in the step is 1.0 to 2.0 equivalents of the nucleoside(s) attached to supports.
[4] The method according to any one of [1] to [3], wherein the activator(s) is selected from the group consisting of 4,5-dicyanoimidazole, 5-(ethylthio)-1H-tetrazole, 5-(benzylthio)-1H-tetrazole and saccharin 1-methylimidazole.
[5] The method according to any one of [1] to [4], wherein the temperature of a solution in the step is 0 to 20°C.

### [Effects of the Invention]

By increasing the used amount of the activator in a method for producing an oligonucleotide, the amount of the nucleoside phosphoramidites remaining in the reaction vessel in the synthesis step can be suppressed, and an oligonucleotide with a favorable purity can be obtained while the used amount of the nucleoside phosphoramidites, which are expensive materials, is reduced. Moreover, also by adjusting the temperature of the solution in each step in the production of an oligonucleotide at a low temperature, the amount of the nucleoside phosphoramidites remaining in the reaction vessel can be suppressed, and an oligonucleotide with a favorable purity can be obtained while the used amount of the nucleoside phosphoramidites, which are expensive materials, is reduced.

Although restriction by any specific theory is not desired, while it is believed that a nucleoside phosphoramidite remains in the reaction vessel because the nucleoside phosphoramidite binds to the hydroxy group formed through unintended removal of the 2-cyanoethyl (CNET) protecting group in the phosphate moiety of the oligonucleotide in the coupling step, resulting in inability of being involved in the chain elongation reaction (the reaction with the hydroxy group at the 5' or 3' end of the nucleoside which is directly or indirectly attached to supports), it is believed that, by increasing the used amount of the activator, binding of the nucleoside phosphoramidite to the hydroxy group of the oligonucleotide formed through the removal of the CNET protecting group is suppressed, and the nucleoside phosphoramidite amount consumed in the reaction vessel decreases, resulting in a reduction in the used amount of the nucleoside phosphoramidite. Moreover, it is believed that, by adjusting the temperature of the solution at a low temperature, the removal of the CNET protecting group in the phosphate moiety of the oligonucleotide is suppressed, and the nucleoside phosphoramidite amount consumed through binding to the hydroxy group of the oligonucleotide formed through the removal of the CNET protecting group decreases, resulting in a reduction in the used amount of the nucleoside phosphoramidite.

### [Brief Description of Drawings]

[Fig. 1] Fig. 1 shows the percentages remaining in the column and the purities of Comparative Example 1, Example 1, Example 2 and Example 3.
[Fig. 2] Fig. 2 shows the percentages remaining in the column and the purities of Comparative Example 2, Example 4, Example 5 and Example 6.
[Fig. 3] Fig. 3 shows the percentages remaining in the column and the purities of Comparative Example 3 and Example 7.
[Fig. 4] Fig. 4 shows the percentages remaining in the column and the purities of Comparative Example 4, Example 8 and Example 9.

### [Description of Embodiments]

The invention is explained in detail below.

Unless otherwise defined in the present specification, all the technical terms and the scientific terms used in the present specification have the same meanings as those generally acknowledged by one skilled in the art. The entire contents of all of the patents, the applications and the other publications and the information that are referred to in the present specification are incorporated in the present specification by reference. When there is a contradiction between a publication that is referred to in the present specification and the descriptions in the present specification, the descriptions in the present specification have priority.

In an aspect, the invention relates to a method for producing an oligonucleotide.

In the invention, the oligonucleotide is produced using the so-called phosphoramidite method, in which a nucleotide is added through condensation reaction of a nucleoside phosphoramidite and a nucleoside, a nucleotide or an oligonucleotide in the presence of a suitable activator.

In the invention, the method for producing an oligonucleotide may include, for example,
(a) a step of removing a protecting group (deprotection) from a protected nucleoside which is directly or indirectly attached to supports and in which the protecting group is bonded to a hydroxy group, a thiol group or an amino group at the 3' position or the 5' position,
(b) a step of binding (coupling) a nucleoside phosphoramidite to the hydroxy group, the thiol group or the amino group at the 3' position or the 5' position of the nucleoside from which the protecting group has been removed and which is directly or indirectly attached to supports in the presence of an activator,
(c) a step of sulfurizing or oxidizing the bond formed in the step (b), and
(d) a step of capping the unbonded hydroxy group, thiol group or amino group at the 3' position or the 5' position of the nucleoside which is directly or indirectly attached to supports.

In the invention, the method for producing an oligonucleotide may include a further step in addition to (a) to (d) above.

In an embodiment of the invention, the method for producing an oligonucleotide includes a step of binding a nucleoside phosphoramidite to a hydroxy group, a thiol group or an amino group at the 3' position or the 5' position of a nucleoside which is directly or indirectly attached to supports in the presence of an activator.

In the invention, the nucleoside refers to a compound in which a nucleoside base and a sugar are bonded and may be a naturally occurring nucleoside, such as adenosine, thymidine, guanosine, cytidine and uridine, or a modified nucleoside. An example of the modified nucleoside is a nucleoside in which the hydroxy group at the 3' position or the 5' position has been substituted with a thiol group or an amino group although the modified nucleoside is not limited thereto. The nucleoside base may be a naturally occurring base, such as adenine, guanine, cytosine, thymine and uracil, or a modified nucleoside base. The sugar moiety of the nucleoside may be naturally occurring deoxyribose or ribose and may have D configuration or L configuration.

In the invention, the nucleotide refers to a compound in which a nucleoside base, a sugar and a phosphate are bonded and may be a naturally occurring nucleotide, such as adenosine triphosphate, thymidine triphosphate, guanosine triphosphate, cytidine triphosphate and uridine triphosphate, or a modified nucleotide. The nucleoside base moiety of the nucleotide may be a naturally occurring base, such as adenine, guanine, cytosine, thymine and uracil, or a modified nucleoside base. The sugar moiety of the nucleoside may be naturally occurring deoxyribose or ribose and may have D configuration or L configuration. The phosphate moiety may be, for example, phosphorothioate, phosphorodithioate, methylphosphonate or methyl phosphate.

In the invention, the oligonucleotide refers to a compound having a structure in which a nucleoside base, a sugar and a phosphate are linked with a phosphodiester bond and includes a naturally occurring oligonucleotide, such as 2'-deoxyribonucleic acid ("DNA" below) and ribonucleic acid ("RNA" below), and a nucleic acid containing a modified sugar moiety, a modified phosphate moiety or a modified nucleobase. The modification of the sugar moiety includes substitution of the ribose ring with a hexose, cyclopentyl or cyclohexyl ring. Alternatively, the D-ribose ring of a naturally occurring nucleic acid may be substituted with an L-ribose ring, or β-anomer of a naturally occurring nucleic acid may be substituted with α-anomer. The oligonucleotide may also contain one or more abasic moieties. The modified phosphate moieties include phosphorothioate, phosphorodithioate, methylphosphonate and methyl phosphate. Such nucleic acid analogues are known to one skilled in the art. An oligonucleotide containing a mixture of two or more of the above can be produced, for example, from an oligonucleotide containing a mixture of deoxyribo- and ribonucleosides, in particular a mixture of a deoxyribonucleoside and a 2'-O-substituted ribonucleoside such as 2'-O-methyl or 2'-O-methoxyethyl ribonucleoside. Examples of the oligonucleotide containing a mixture of nucleosides include ribozymes.

In the invention, the nucleoside phosphoramidite refers to a nucleoside formed into a derivative by an amidite. In the invention, in the nucleoside phosphoramidite, either of the hydroxy group at the 3' position or the hydroxy group at the 5' position of a nucleoside has been formed into a phosphoramidite, and a protecting group is bonded to the other.

The amidite formation can be conducted, for example, using 1H-tetrazole as an activator by reacting 2-cyanoethyl-N,N,N',N'- tetraisopropylphosphordiamidite with an appropriately protected nucleoside.

The nucleoside phosphoramidite may be a monomer or an oligomer such as 2-mer to 24-mer.

In the invention, the activator refers to an agent which activates the nucleoside phosphoramidite, is used for reacting a nucleoside, a nucleotide or an oligonucleotide and is also called a coupling agent. In the invention, an activator which is generally used in the phosphoramidite method can be used. Examples of the activator used in the invention include 4,5-dicyanoimidazole, 5-(ethylthio)-1H-tetrazole, 5-(benzylthio)-1H-tetrazole, saccharin 1-methylimidazole and the like although the activator is not limited thereto, and 4,5-dicyanoimidazole is preferable.

In the invention, the "nucleoside which is directly attached to supports" refers to the nucleoside moiety of a compound in which a nucleoside or a nucleotide is bonded to the reactive sites on supports (the compound moiety in which a nucleoside base and a sugar are bonded), and the "nucleoside which is indirectly attached to supports" refers to the nucleoside moiety in which a nucleotide is bonded to the reactive sites on supports through a compound such as a polynucleotide (the compound moiety formed through binding of a nucleoside base and a sugar).

In an embodiment of the invention, the amount of the activator used in the step of binding a nucleoside phosphoramidite to a hydroxy group, a thiol group or an amino group at the 3' position or the 5' position of a nucleoside which is directly or indirectly attached to supports in the presence of an activator is, for example, 10.0 to 15.0 equivalents of the amount of the nucleoside phosphoramidite used in the step. Specifically, the amount is 10.0 equivalents, 10.5 equivalents, 11.0 equivalents, 11.5 equivalents, 12.0 equivalents, 12.5 equivalents, 13.0 equivalents, 13.5 equivalents, 14.0 equivalents, 14.5 equivalents or 15.0 equivalents and may be in the range between any two of the numerical values.

In an embodiment of the invention, the amount of the activator used in the step of binding a nucleoside phosphoramidite to a hydroxy group, a thiol group or an amino group at the 3' position or the 5' position of a nucleoside which is directly or indirectly attached to supports in the presence of an activator is not particularly limited but is, for example, preferably 10.0 to 25.0 equivalents of the nucleosides attached to supports. Specifically, the amount is 10.0 equivalents, 10.5 equivalents, 11.0 equivalents, 11.5 equivalents, 12.0 equivalents, 12.5 equivalents, 13.0 equivalents, 13.5 equivalents, 14.0 equivalents, 14.5 equivalents, 15.0 equivalents, 15.5 equivalents, 16.0 equivalents, 16.5 equivalents, 17.0 equivalents, 17.5 equivalents, 18.0 equivalents, 18.5 equivalents, 19.0 equivalents, 19.5 equivalents, 20.0 equivalents, 20.5 equivalents, 21.0 equivalents, 21.5 equivalents, 22.0 equivalents, 22.5 equivalents, 23.0 equivalents, 23.5 equivalents, 24.0 equivalents, 24.5 equivalents or 25.0 equivalents and may be in the range between any two of the numerical values.

In an embodiment of the invention, the amount of the nucleoside phosphoramidite used in the step of binding a nucleoside phosphoramidite to a hydroxy group, a thiol group or an amino group at the 3' position or the 5' position of a nucleoside which is directly or indirectly attached to supports in the presence of an activator is not particularly limited but is, for example, preferably 1.0 to 2.0 equivalents of the nucleosides attached to supports. Specifically, the amount is 1.0 equivalent, 1.1 equivalents, 1.2 equivalents, 1.3 equivalents, 1.4 equivalents, 1.5 equivalents, 1.6 equivalents, 1.7 equivalents, 1.8 equivalents, 1.9 equivalents or 2.0 equivalents and may be in the range between any two of the numerical values.

In an embodiment of the invention, the temperature of the solution in the step of binding a nucleoside phosphoramidite to a hydroxy group, a thiol group or an amino group at the 3' position or the 5' position of a nucleoside which is directly or indirectly attached to supports in the presence of an activator is not particularly limited but is, for example, preferably 0 to 30°C, more preferably 0 to 20°C, further preferably 5 to 20°C. Specifically, the temperature is 0°C, 1°C, 2°C, 3°C, 4°C, 5°C, 6°C, 7°C, 8°C, 9°C, 10°C, 11°C, 12°C, 13°C, 14°C, 15°C, 16°C, 17°C, 18°C, 19°C, 20°C, 21°C, 22°C, 23°C, 24°C, 25°C, 26°C, 27°C, 28°C, 29°C or 30°C and may be in the range between any two of the numerical values.

In an embodiment of the invention, in the method for producing an oligonucleotide which includes
(a) a step of removing a protecting group from a protected nucleoside which is directly or indirectly attached to supports and in which the protecting group is bonded to a hydroxy group, a thiol group or an amino group at the 3' position or the 5' position,
(b) a step of binding a nucleoside phosphoramidite to the hydroxy group, the thiol group or the amino group at the 3' position or the 5' position of the nucleoside from which the protecting group has been removed and which is directly or indirectly attached to supports in the presence of an activator,
(c) a step of sulfurizing or oxidizing the bond formed in the step (b), and
(d) a step of capping the unbonded hydroxy group, thiol group or amino group at the 3' position or the 5' position of the nucleoside which is directly or indirectly attached to supports,
the temperature of the solution in any step of (a) to (d) is 0 to 30°C, preferably 0 to 20°C, more preferably 5 to 20°C. Specifically, the temperature is 0°C, 1°C, 2°C, 3°C, 4°C, 5°C, 6°C, 7°C, 8°C, 9°C, 10°C, 11°C, 12°C, 13°C, 14°C, 15°C, 16°C, 17°C, 18°C, 19°C, 20°C, 21°C, 22°C, 23°C, 24°C, 25°C, 26°C, 27°C, 28°C, 29°C or 30°C and may be in the range between any two of the numerical values.

Here, the method for regulating the temperature of the solution is a method of feeding the solution which is regulated to a desired temperature in advance to each step, a method of feeding the solution in each step and then regulating to a desired temperature by cooling the reaction vessel or the like, a method of providing a pipe in the reaction vessel and causing a refrigerant or the like to flow in the pipe to regulate to a desired temperature or another method. Moreover, in the invention, the temperature of the solution which is regulated to a desired temperature in advance during feeding to each step is called a feeding liquid temperature.

In the present specification, the amount of the nucleoside phosphoramidite remaining in the reaction vessel refers to the amount of the nucleoside phosphoramidite that remains in the reaction vessel in the synthesis step of the oligonucleotide, for example, based on the theory explained above or the like. The amount of the phosphoramidite remaining in the reaction vessel can be measured, for example, by collecting the liquid waste of the oxidation step or the sulfurization step in each synthesis cycle and any wash solution after the coupling reaction during the synthesis cycle and analyzing the amount of the DMTr (4,4'-dimethoxytriphenylmethyl group) protecting group of the nucleoside phosphoramidite by HPLC.

### [Examples]

Although the invention is explained in more detail referring to the following examples, the examples show particular specific examples of the invention, and the invention is not limited thereto.

### (1) Synthesis of DNA Oligonucleotide

Porous resin beads (NittoPhase (registered trademark) HL UnyLinker350) were put into a synthesis column (volume of 12.6 ml) at a synthesis scale (the total reactive sites on the beads) of 480 µmol and set in an AKTA oligopilot plus 100 synthesizer (manufactured by Cytiva), and nucleoside phosphoramidites and 4,5-dicyanoimidazole (DCI) as an activator in the amounts shown in Table 1 were introduced. The coupling reaction (reaction time: five minutes) was conducted under the conditions shown in Table 1. The entire activator was dissolved in acetonitrile and adjusted to 0.7 M. The other synthesis reagents used were 3% DCA in toluene as a deprotection agent, xanthane hydride in pyridine adjusted to 0.2 M as a sulfurization agent, a mixed solution of lutidine, N-methylimidazole and acetic anhydride in acetonitrile as a capping agent and TBA in acetonitrile (at a ratio of 2:8) as an amine wash reaction solution. A 24-mer DNA oligonucleotide (5'-TCGACGTATTGACGTATTGACGTA-3', the phosphite esters were completely sulfurized: SEQ ID NO: 1) was synthesized, and the DMTr protecting group at the end was removed. After the synthesis, the porous resin beads to which the DNA oligonucleotide was bonded were dried. Then, the porous resin beads were treated with ammonium water, and the DNA oligonucleotide was cleaved from the porous resin beads. The base amino group was deprotected, and a filtrate in which the DNA oligonucleotide was dissolved was obtained.

### (2) Measurement of Purity of Synthesized DNA Oligonucleotide

The oligonucleotide sample filtrate which was adjusted to 5 OD was measured by high-performance liquid chromatography (HPLC) under the following conditions. Here, the total sum of the peak area from the detection of the main component to about 10 minutes was set as 100%, and the peak area (%) of the main component was regarded as the synthesis purity (full-length: area %).

Column: manufactured by Waters Corporation, ACQUITY UPLC Oligonucleotide BEH C18 Column, 130 A, 1.7 µm, 2.1 mm × 100 mm.
UV detection: 260 nm
Mobile phase A: aqueous 400 mM HFIP/15 mM TEA solution
Mobile phase B: methanol
Column temperature: 60°C

### (3) Measurement of Amount of Nucleoside Phosphoramidites Remaining in Column

Because the nucleoside phosphoramidites bonded to the phosphate moiety of the oligonucleotide were removed by the sulfurization agent and discharged with the liquid waste in the sulfurization step, all the liquid wastes of the sulfurization step in the synthesis of the 24-mer DNA, namely in the 24 synthesis cycles in total, were collected. Here, after the coupling reaction of the 24th synthesis cycle, the inside of the column was washed with five times the column volume (63 ml) of acetonitrile for sufficient washing, and this was also collected as the liquid waste. The amount of the nucleoside phosphoramidites was estimated from the absolute quantity of the DMTr protecting group of the nucleoside phosphoramidites. The nucleoside phosphoramidites were hydrolyzed and diluted with 0.1 M para-toluenesulfonic acid monohydrate (pTSA) in acetonitrile solution to remove DMTr protecting group. The absolute quantity of the DMTr protecting group was measured by HPLC under the following conditions.
Column: manufactured by Waters Corporation, Atlantis T3, 130 A, 3.0 µm, 2.1 mm × 150 mm
MS detection: ESI-Positive. m/z303
Mobile phase A: aqueous 0.1% formic acid solution
Mobile phase B: acetonitrile
Column temperature: 40°C

The percentage of the nucleoside phosphoramidites remaining in the column was calculated by dividing the absolute quantity of DMTr in the liquid wastes (corresponding to the bonded nucleoside phosphoramidite amount) by the amount of the nucleoside phosphoramidites introduced to the reaction (synthesis scale × nucleoside phosphoramidite equivalent).

### (4) Results

The results are shown in Table 1.

### [Table 1]

**Table 1**

| | Amidite | Activator | Activator / Amldlte | Feeding liquid temperature | | | | Results | | |
|---|---|---|---|---|---|---|---|---|---|---|
| | Introduction amount | Introduction amount | | Deprotecting | Coupling | Sulfurating | Capping | Percentage remaining in the column | | Purity |
| | Equivalent | Equivalent | times | °C | °C | °C | °C | % | % based on a Comparative Example | % |
| Example 1 | 1.7 | 17.9 | 10.5 | 22.5 | 22.5 | 22.5 | 22.5 | 26.7 | 0.93 | 86.8 |
| Example 2 | 1.7 | 23.8 | 14 | 22.5 | 22.5 | 22.5 | 22.5 | 26.0 | 0.91 | 87.8 |
| Example 3 | 1.7 | 23.8 | 14 | 15 | 15 | 15 | 15 | 22.0 | 0.77 | 90.8 |
| Comparative Example 1 | 1.7 | 6.0 | 3.5 | 22.5 | 22.5 | 22.5 | 22.5 | 28.6 | - | 84.7 |
| Example 4 | 1.2 | 12.6 | 10.5 | 22.5 | 22.5 | 22.5 | 22.5 | 18.8 | 0.94 | 85.3 |
| Example5 | 1.2 | 16.8 | 14 | 22.5 | 22.5 | 22.5 | 22.5 | 18.7 | 0.94 | 85.4 |
| Example6 | 1.2 | 16.8 | 14 | 15 | 15 | 15 | 15 | 16.7 | 0.84 | 87.2 |
| Comparative Example 2 | 1.2 | 4.2 | 3.5 | 22.5 | 22.5 | 22.5 | 22.5 | 19.9 | - | 80.2 |

Regarding the % based on a Comparative Example, the values of Examples 1 to 3 show the remaining percentages of Examples 1 to 3, where the percentage remaining in the column of Comparative Example 1 is regarded as 1, and the values of Examples 4 to 6 show the remaining percentages of Examples 4 to 6, where the percentage remaining in the column of Comparative Example 2 is regarded as 1. When the % based on the Comparative Example is less than 1, this shows that the amount remaining in the column is suppressed compared to that of the Comparative Example.

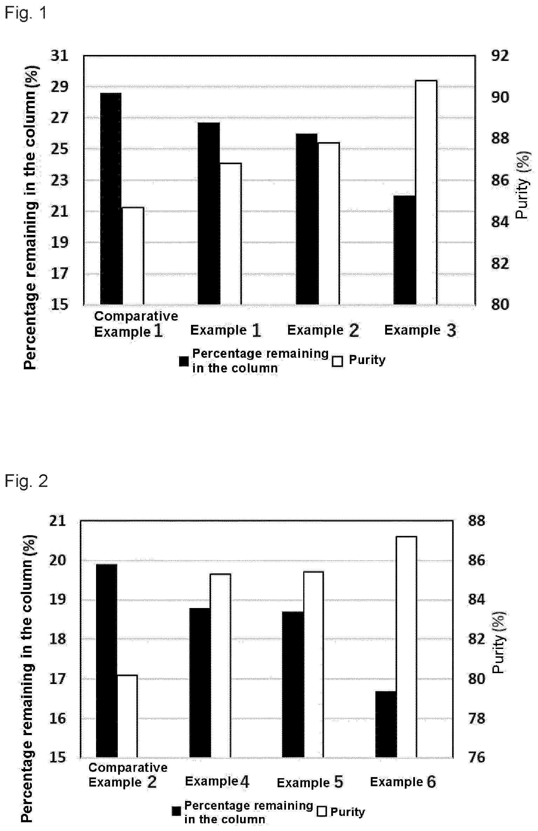

The percentages remaining in the column and the purities of Comparative Example 1, Example 1 and Example 2, in which 1.7 equivalents of the nucleoside phosphoramidites were used and the amounts of the activator used were 3.5 times, 10.5 times and 14 times, respectively, based on the amount of the nucleoside phosphoramidites, and Example 3, in which the amount of the activator used was 14 times the amount of the nucleoside phosphoramidites and the feeding liquid temperature was 15°C, are shown in Fig. 1. When the used amount of the activator increased, the percentage of the nucleoside phosphoramidites remaining in the column decreased, and the purity of the synthesized DNA oligonucleotide increased. Moreover, by decreasing the feeding liquid temperature to 15°C as in Example 3, the percentage of the nucleoside phosphoramidites remaining in the column decreased further, and the purity of the synthesized DNA oligonucleotide increased.

The percentages remaining in the column and the purities of Comparative Example 2, Example 4 and Example 5, in which 1.2 equivalents of the nucleoside phosphoramidites were used and the amounts of the activator used were 3.5 times, 10.5 times and 14 times, respectively, based on the amount of the nucleoside phosphoramidites, and Example 6, in which the amount of the activator used was 14 times the amount of the nucleoside phosphoramidites and the feeding liquid temperature was 15°C, are shown in Fig. 2. When the used amount of the activator increased, the percentage of the nucleoside phosphoramidites remaining in the column decreased, and the purity of the synthesized DNA oligonucleotide increased. Moreover, by decreasing the feeding liquid temperature to 15°C as in Example 6, the percentage of the nucleoside phosphoramidites remaining in the column decreased further, and the purity of the synthesized DNA oligonucleotide increased.

### (5) Synthesis of RNA Oligonucleotide

Porous resin beads (NittoPhase (registered trademark) HL 250-2'OMeA) were put into a synthesis column (volume of 12.6 ml) at a synthesis scale (the total reactive sites on the beads) of 352 µmol and set in an AKTA oligopilot plus 100 synthesizer (manufactured by Cytiva), and nucleoside phosphoramidites and 5-(ethylthio)-1H-tetrazole (ETT) as an activator in the amounts shown in Table 2 were introduced. The coupling reaction (reaction time: 10 minutes) was conducted under the conditions shown in Table 2. The entire activator was dissolved in acetonitrile and adjusted to 0.6 M. The other synthesis reagents used were 3% DCA in toluene as a deprotection agent, an iodine solution (0.05 mol/L: the solvent was at a ratio of pyridine:water (9:1)) as an oxidizer, a mixed solution of lutidine, N-methylimidazole and acetic anhydride in acetonitrile as a capping agent and TBA in acetonitrile (at a ratio of 2:8) as an amine wash reaction solution. A 24-mer 2'OMe RNA oligonucleotide (5'-UCGACGUAUUGACGUAUUGACGUA-3', the phosphite esters were completely oxidized: SEQ ID NO: 2) was synthesized, and the DMTr protecting group at the end was removed. The porous resin beads to which the RNA oligonucleotide was bonded were dried. Then, the porous resin beads were treated with ammonium water, and the RNA oligonucleotide was cleaved from the porous resin beads. The base amino group was deprotected, and a filtrate in which the 2'OMe RNA oligonucleotide was dissolved was obtained.

### (6) Measurement of Purity of Synthesized RNA Oligonucleotide

The oligonucleotide sample filtrate which was adjusted to 5 OD was measured by high-performance liquid chromatography (HPLC) under the following conditions. Here, the total sum of the peak area from the detection of the main component to about 10 minutes was set as 100%, and the peak area (%) of the main component was regarded as the synthesis purity (full-length: area %).
Column: manufactured by Waters Corporation, ACQUITY UPLC Oligonucleotide BEH C18 Column, 130 A, 1.7 µm, 2.1 mm × 100 mm.
UV detection: 260 nm
Mobile phase A: aqueous 400 mM HFIP/15 mM TEA solution
Mobile phase B: methanol
Column temperature: 60°C

### (7) Measurement of Amount of Nucleoside Phosphoramidites Remaining in Column

Because the nucleoside phosphoramidites bonded to the phosphate moiety of the oligonucleotide were removed by the oxidizer and discharged with the liquid waste in the oxidation step, all the liquid wastes of the oxidation step in the synthesis of the 24-mer 2'OMe RNA, namely in the 24 synthesis cycles in total, were collected. Here, after the coupling reaction of the 24th synthesis cycle, the inside of the column was washed with five times the column volume (63 ml) of acetonitrile for sufficient washing, and this was also collected as the liquid waste. The amount of the nucleoside phosphoramidites was estimated from the absolute quantity of the DMTr protecting group of the nucleoside phosphoramidites. The nucleoside phosphoramidites were hydrolyzed and diluted with 0.1 M para-toluenesulfonic acid monohydrate (pTSA) in acetonitrile solution to remove DMTr protecting group. The absolute quantity of the DMTr protecting group was measured by HPLC under the following conditions.
Column: manufactured by Waters Corporation, Atlantis T3, 130 A, 3.0 µm, 2.1 mm × 150 mm
MS detection: ESI-Positive. m/z303
Mobile phase A: aqueous 0.1% formic acid solution
Mobile phase B: acetonitrile
Column temperature: 40°C

The percentage of the nucleoside phosphoramidites remaining in the column was calculated by dividing the absolute quantity of DMTr in the liquid wastes (corresponding to the bonded nucleoside phosphoramidite amount) by the amount of the nucleoside phosphoramidites introduced to the reaction (synthesis scale × nucleoside phosphoramidite equivalent).

### (8) Results

The results are shown in Table 2.

### [Table 2]

**Table 2**

| | Amidite | Activator | Activator / Amidite | Feeding liquid temperature | | | | Results | | |
|---|---|---|---|---|---|---|---|---|---|---|
| | Introduction amount | Introduction amount | | Deprotecting | Coupling | Sulfurating | Capping | Percentage remaining in the column | | Purity |
| | Equivalent | Equivalent | times | °C | °C | °C | °C | % | % based on a Comparative Example | % |
| Example 7 | 2 | 24 | 12.0 | 22.5 | 22.5 | 22.5 | 22.5 | 12.7 | 0.63 | 86.7 |
| Comparative Example 3 | 2 | 6.6 | 3.3 | 22.5 | 22.5 | 22.5 | 22.5 | 20.3 | - | 85.5 |
| Example 8 | 1.4 | 16.8 | 12.0 | 22.5 | 22.5 | 22.5 | 22.5 | 10.7 | 0.85 | 85.1 |
| Example 9 | 1.4 | 16.8 | 12.0 | 15 | 15 | 15 | 15 | 7.7 | 0.61 | 87.6 |
| Comparative Example 4 | 1.4 | 4.6 | 3.3 | 22.5 | 22.5 | 22.5 | 22.5 | 12.6 | - | 80.0 |

Regarding the % based on a Comparative Example, the value of Example 7 shows the remaining percentage of Example 7, where the percentage remaining in the column of Comparative Example 3 is regarded as 1, and the values of Examples 8 and 9 show the remaining percentages of Examples 8 and 9, where the percentage remaining in the column of Comparative Example 4 is regarded as 1. When the % based on the Comparative Example is less than 1, this shows that the amount remaining in the column is suppressed compared to that of the Comparative Example.

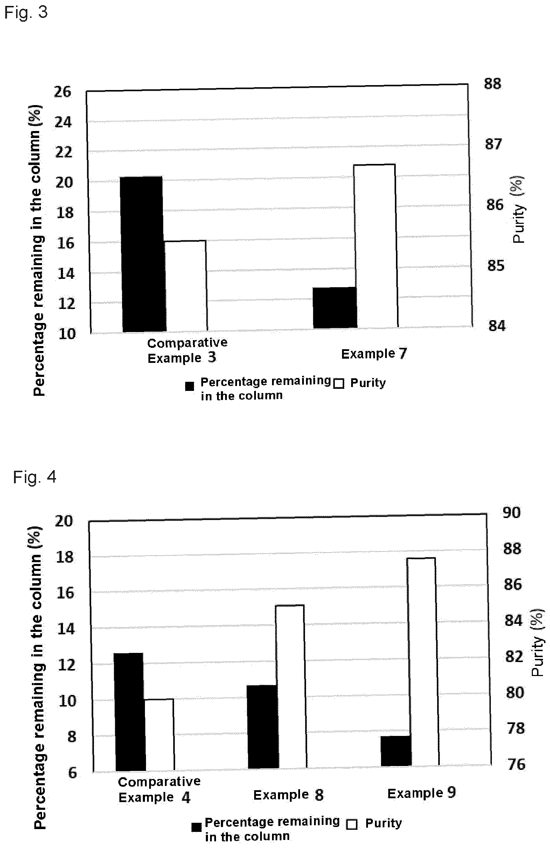

The percentages remaining in the column and the purities of Comparative Example 3 and Example 7, in which 2.0 equivalents of the nucleoside phosphoramidites were used, the feeding liquid temperature was 22.5°C and the amounts of the activator used were 3.3 times and 12.0 times, respectively, based on the amount of the nucleoside phosphoramidites, are shown in Fig. 3. The percentage of the synthesized RNA oligonucleotide remaining in the column decreased to 12.7% when the amount of the activator was 12.0 times the amount of the nucleoside phosphoramidites, and the purity of the RNA oligonucleotide increased.

The percentages remaining in the column and the purities of Comparative Example 4 and Example 8, in which 1.4 equivalents of the nucleoside phosphoramidites were used, the feeding liquid temperature was 22.5°C and the amounts of the activator used were 3.3 times and 12.0 times, respectively, based on the amount of the nucleoside phosphoramidites, and Example 9, in which 1.4 equivalents of the nucleoside phosphoramidites were used, the amount of the activator used was 12 times the amount of the nucleoside phosphoramidites and the feeding liquid temperature was 15°C, are shown in Fig. 4. Also in Fig. 4, when the amount of the activator was increased to 12.0 times the amount of the nucleoside phosphoramidites, the percentage remaining in the column decreased, and the purity of the synthesized RNA oligonucleotide was a high value. In Example 9, the percentage of nucleoside phosphoramidites remaining in the column further decreased, and the purity was a higher value. Therefore, it was found that, in Example 9 as compared to Comparative Example 3, also when modified RNA nucleoside phosphoramidites were used, the purity could be increased by decreasing the percentage remaining in the column despite the significant reduction in the introduction amount of the RNA nucleoside phosphoramidites.

## Claims

1. A method for producing an oligonucleotide comprising a step of binding a nucleoside phosphoramidite(s) to a hydroxy group, a thiol group or an amino group at the 3' position or the 5' position of a nucleoside(s) which is directly or indirectly attached to supports in the presence of an activator(s),
wherein the amount of the activator(s) used in the step is 10.0 to 15.0 equivalents of the amount of the nucleoside phosphoramidite(s) used in the step.

2. The method according to claim 1, wherein the amount of the activator(s) used in the step is 10.0 to 25.0 equivalents of the nucleoside(s) attached to supports.

3. The method according to claim 1 or 2, wherein the amount of the nucleoside phosphoramidite(s) used in the step is 1.0 to 2.0 equivalents of the nucleoside(s) attached to supports.

4. The method according to any one of claims 1 to 3, wherein the activator(s) is selected from the group consisting of 4,5-dicyanoimidazole, 5-(ethylthio)-1H-tetrazole, 5-(benzylthio)-1H-tetrazole and saccharin 1-methylimidazole.

5. The method according to any one of claims 1 to 4, wherein the temperature of a solution in the step is 0 to 20°C.
